(19) Europäisches Patentamt / European Patent Office / Office européen des brevets

(11) **EP 4 596 699 A1**

(12) **EUROPEAN PATENT APPLICATION**
published in accordance with Art. 153(4) EPC

(43) Date of publication:
06.08.2025 Bulletin 2025/32

(21) Application number: 23873039.4

(22) Date of filing: 25.09.2023

(51) International Patent Classification (IPC):
*C12N 15/85* (2006.01)   *C07K 14/47* (2006.01)
*C12N 15/63* (2006.01)   *C12N 5/073* (2010.01)

(52) Cooperative Patent Classification (CPC):
C07K 14/47; C12N 5/06; C12N 15/63; C12N 15/85

(86) International application number:
PCT/KR2023/014695

(87) International publication number:
WO 2024/071923 (04.04.2024 Gazette 2024/14)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 29.09.2022 KR 20220124120

(71) Applicant: Kainos Medicine, Inc.
Seongnam-si, Gyeonggi-do 13488 (KR)

(72) Inventors:
• KIM, Eunhee
Seoul 06331 (KR)
• HAN, Jeong-Hoon
Seongnam-si, Gyeonggi-do 13488 (KR)
• PARK, Daeho
Seongnam-si, Gyeonggi-do 13488 (KR)
• JUNG, Bum Jun
Seongnam-si, Gyeonggi-do 13488 (KR)

(74) Representative: Dehns
10 Old Bailey
London EC4M 7NG (GB)

Remarks:
The complete document including Reference
Table(s) and the Sequence Listing(s) can be
downloaded from the EPO website

(54) **CELL LINE FOR PRODUCING FAF1 EXOSOMES IN HIGH YIELD, AND PREPARATION METHOD THEREFOR**

(57) According to an aspect of the technology disclosed in the present application, the present invention relates to a cell line in which exosomes loaded with FAF 1 protein are stably expressed, and relates to: a cell line, which can produce, in a high yield, exosomes loaded with FAF 1 protein and selectively modulate the expression of FAF1 protein; a method for preparing the cell line; and a method for producing exosomes loaded with FAF 1 protein.

[Fig. 5]

EP 4 596 699 A1

**Description**

**Technical Field**

The present invention relates to a cell line capable of mass-producing a FAF1-loaded exosome and expressing FAF1 only when necessary, and stably expressing the exosome, a method for preparing the cell line, and a method for producing the FAF1-loaded exosome.

**Background Art**

Tumor cells are known to have resistance to apoptosis due to genetic mutations associated with apoptosis. There are several types of signal pathways and receptors associated with apoptosis, but among them, Fas (also known as Fas receptor, CD95, Apo1, or TNFRSF6) is one of the receptors having excellent apoptosis ability. In addition, it has been reported that the expression level of Fas-associated Factor 1 (FAF1), a protein associated with the Fas pathway, is decreased in various cancers including lung cancer, colon cancer, liver cancer, prostate cancer, brain cancer, and breast cancer (XIE, Feng, et al. FAF1 phosphorylation by AKT accumulates TGF-B type II receptor and drives breast cancer metastasis. Nature communications, 2017, 8.1: 1-16).

Meanwhile, extracellular vesicles refer to nano-sized vesicles that cells secrete to the external environment for intercellular information exchange. The extracellular vesicle is referred to as various names, such as exosome, microvesicle, ectosome, microparticle, membrane vesicle, nanovesicle, outer membrane vesicle, and the like, based on origin, secretion mechanism, size, and the like, and include various substances exhibiting biological activity, such as proteins, lipids, nucleic acids, and metabolites.

Among extracellular vesicles, exosomes have recently attracted attention in the field of disease diagnosis and treatment due to various advantages. Since exosomes are not living cells, the exosomes may be stored and transported for a long period of time, and there is little risk of tumor generation due to no cell division. In addition, since the exosomes are cell-derived materials, the exosomes have little immunogenicity and have the same membrane topology as cells, and thus may easily deliver drugs/vaccines loaded as cargo materials to specific cells or tissues. Therefore, the exosomes have been recently used in the development of a medicament for treating a tumor. For example, Korean Patent No. 10-2053065 relates to a pH-sensitive exosome composition using hyaluronic acid and doxorubicin, and discloses a method for preparing a pH-sensitive exosome using doxorubicin and a polymer chemically combining hyaluronic acid and 3-diethylamino propylamine, and a cancer cell killing effect of the exosome. In addition, Korean Patent Laid-open Publication No. 10-2018-0078173 relates to a novel exosome anti-cancer agent, and discloses a recombinant exosome in which a receptor tyrosine kinase and SIRP (i.e., phagocytosis promoting protein), are presented on the surface thereof and a recombinant exosome including an asparaginase, a protein toxin, an antibody specific to a cancer antigen or a fragment of the antibody, a tumor suppressor gene or an anti-angiogenic factor, and the like, which are anti-cancer proteins.

Since the amount of exosomes naturally secreted by cells is very limited, the development of cell lines that mass-produce exosomes at a high yield is required in order to use exosomes for actual tumor treatment purposes.

On the other hand, when the protein generated by a cell line is a protein inducing apoptosis, the protein adversely affects cell survival and proliferation, and thus the protein productivity may deteriorate as it approaches the second half of the culture. Accordingly, there is a need to develop a cell line and an exosome production method that may mass-produce exosomes loaded with proteins inducing apoptosis but do not affect the survival and proliferation of cells producing exosomes.

**Disclosure of the Invention**

**Technical Problem**

The purpose of the present invention is to provide a cell line which stably expresses an exosome loaded with a FAF1 protein, a method for preparing the cell line, and a method for mass-producing the exosome loaded with the FAF1 protein using the cell line.

**Solution to Problem**

In an aspect of the present invention, the present invention provides a cell line stably expressing an exosome loaded with a FAF 1 protein.

In another aspect of the present invention, the present invention provides a method for preparing a cell line stably expressing an exosome loaded with a FAF1 protein, the method including the steps of: a) transducing a vector including a gene encoding the FAF 1 protein and a promoter regulating the expression of the gene into an immune cell, a stem cell, a somatic cell, a plant cell, a bacterial cells an yeast cell, a mammalian cell or a tumor cell to obtain a transformed cell; b) culturing the transformed cells; and c) screening a cell stably expressing the exosome loaded with the FAF 1 protein in the cultured cells.

In another aspect of the present invention, the present invention provides a method for producing an exosome loaded with a FAF 1 protein, the method including the steps of: a) culturing a cell line stably expressing the exosome loaded with the FAF1 protein; and b) isolating the exosome loaded with the FAF 1 protein from the cell line culture medium.

**Advantageous Effects of Invention**

According to an embodiment of the present invention, a cell line stably expressing an exosome loaded with a FAF1 protein may produce FAF1 protein loaded exosomes with high yield, and may mass-produce exosomes loaded with the FAF1 protein by being less affected by apoptosis induced by the FAF1 protein.

**Brief Description of Drawings**

FIG. 1 shows western blotting results showing results of inducing FAF 1 expression with doxycycline in cells in which pTetOne-FAF1 transduction was confirmed.

FIG. 2 is a diagram showing viabilities of F5-3 cell line, F5-4 cell line, and F3-1J cell line compared to Expi293F cell line.

FIG. 3 is a diagram showing doubling times of the F5-3 cell line, F5-4 cell line, and F3-1J cell line compared to the Expi293F cell line.

FIG. 4 shows diagrams showing changes in the expression of exosome-related markers in the Expi293F cell line and the F5-3 cell line depending on the presence or absence of doxycycline.

FIG. 5 shows the produced amount of exosomes, the produced amount of FAF1, and the purity of FAF1 in the exosomes in the HEK293 cell line, the Expi293F cell line, and the F5-3 cell line depending on the presence or absence of doxycycline.

**Best Mode for Carrying out the Invention**

Hereinafter, the present invention will be described in detail.

An aspect of the present invention relates to a cell line stably expressing an exosome loaded with a FAF1 protein.

As used herein, the term "FAF1" refers to Fas-associated Factor 1, which is involved in cell proliferation by mediating cell necrosis through JNK dependent mitochondrial dysfunction and negative-controlling Aurora-A to suppress G2/M phase during a cell cycle. In addition, the FAF1 binds to a ubiquitinated protein and the Valosin Containing Protein (VCP) and is involved in the ubiquitin-proteasome pathway to regulate degradation of the protein, and unnecessary FAF1 is ubiquitinated through Parkin and degraded through the proteasome pathway.

FAF1 is involved in various biochemical processes including cell death, inflammation, cell proliferation, and protein homeostasis by activating various pathways. In particular, FAF1 is a tumor suppressor which induces cell death by forming a Fas-death inducing signaling complex (Fas-DISC), which acts as a tumor suppressor through NF-κB inhibition, and also suppresses tumor metastasis through TGF-β signal.

As used herein, the term "apoptosis" is a kind of programmed cell death that may occur in multicellular organisms. The apoptosis leads to cell death due to changes in cell morphology and biochemical changes inside the cell. This process ends with cell swelling and cracking, cell membrane changes, chromatin condensation and chromosome cleavage, and the cell being engulfed by a phagocyte. In contrast to necrosis, which is cell death caused by acute cellular injury, apoptosis does not harm organisms but confers advantages on the life cycle thereof. The formation of fingers and toes in the process of differentiation of human embryo is a representative example of apoptosis. In addition, apoptosis is an important mechanism in cell replacement, tissue remodeling, and removal of damaged cells.

As used herein, The FAF1 protein may include the amino acid sequence of Uniprot ID Q9UNN5 or the amino acid sequence of SEQ ID NO: 1, and preferably may be a polypeptide consisting of the amino acid sequence of SEQ ID NO: 1.

As used herein, the term "exosome" refers to a vesicle made of two layers of phospholipid membranes secreted by a cell. It is known that exosomes perform cell-to-cell signaling, form disease-specific nucleic acids and proteins, and release them into body fluids, and play an important role in intercellular communication through intercellular delivery of specific repertoires of nucleic acids, proteins, and lipids which are important for homeostasis. For example, exosomes are involved in basic physiological processes such as neurotransmission, antigen presentation, immune response, organ development, and reproductive ability, and also in some pathological disorders including cancer progression, cardiovascular disease, inflammation, and prion transmission.

In addition, exosomes are secreted into the extracellular environment after a late endosome called a multivesicular body (MVB) containing intraluminal vesicles (ILVs) fuses with the cell membrane. The MVB may fuse with the cell membrane to release ILVs, or on the other hand, it may fuse with lysosome to degrade the contents. The materials present in the exosomes are not similar to the composition of cytoplasmic materials, and the exosomes may selectively contain RNAs and proteins.

Although it has been reported that the production of exosomes is dependent on or independent of endosomal sorting complex required for transport (ESCRT), it is difficult to discover the exact mechanism. Cells secrete proteins in which signal peptides are present through the endoplasmic reticulum-Golgi complex. The vesicles containing the proteins in which the signal peptides are present move toward the cell membrane, fuse with the cell membrane, and send the protein out of the cell. However, proteins without the signal peptides are possible to be secreted through an alternative non-classical secretory pathway. When the proteins without signal peptides are secreted, the proteins are secreted either in the absence or presence of vesicles, and although the exact mechanism of the non-vesicular secretory pathway is unknown, some proteins are secreted via membrane pores and ATP-binding cassette transporters. Vesicular secretion is carried out via extracellular vesicles, including exosomes, and is carried out via vesicles with various sizes.

The exosomes may have an average diameter of 50 nm to 300 nm, but the present invention is not limited thereto.

In addition, in another aspect of the present invention, the cell line stably expressing the exosome loaded with the FAF1 protein may be a cell line transformed with a vector including a gene encoding the FAF 1 protein and a promoter regulating the expression of the gene. In this case, the FAF 1 protein is the same as described above.

Furthermore, a vector including a linear puromycin marker gene may be used during transformation for preparing a cell line stably expressing an exosomes loaded with a FAF 1 protein. The vector including the linear puromycin marker gene may include a puromycin marker gene, an SV40 promoter, and an SV40 polyadenylation signal, and may preferably be a linear puromycin marker (Catalog No. 631626) included in the Tet-One™ Inducible Expression System of Takara Bio Inc. (Japan).

As used herein, the term "transformation" refers to a molecular biological technique in which a vector including an exogenous gene different from that of the original cell is introduced into the cell and the exogenous gene binds to the DNA present in the original cell, thereby altering the genetic character of the cell.

As used herein, the term "vector" refers to a means for expressing a gene of interest in a host cell. For example, the vector may be a chromosomal, episomal, plasmid vector, or single-stranded or double-stranded RNA or DNA viral vector. In addition, the vector includes a phagemid vector, a cosmid vector, a bacteriophage vector, and a viral vector such as an adenoviral vector, a retroviral vector, and an adeno-associated viral vector. A vector that may be used as the vector may be produced by engineering a plasmid (*e.g.,* pSC101, pGV1106, pACYC177, ColE1, pKT230, pME290, pBR322, pUC8/9, pUC6, pBD9, pHC79, pIJ61, pLAFR1, pHV14, pGEX series, pET series, pUC19, *etc.*), a phage (*e.g.,* λgt4λB, λCharon, M13, *etc.*), or a virus (*e.g.,* CMV, SV40, *etc.*), which are often used in the art, and may preferably be PRSET A. In addition, the vector may be introduced into the cell by a well-known technique for introducing DNA and RNA into the cell.

In addition, in an aspect of the present invention, the promoter that regulates the expression of the gene encoding the protein may be a promoter operated by a protein synthesis inhibitor. The protein synthesis inhibitor may be selected from the group consisting of aminoglycoside antibiotics, tetracycline antibiotics, macrolide antibiotics, lincosamide antibiotics, streptogramin antibiotics, pleuromutilin antibiotics, phenicol antibiotics, fusidic acid antibiotics, oxazolidinone antibiotics, anisomycin antibiotics, edeine antibiotics, pactamycin antibiotics, puromycin antibiotics, cyclohexamide, aurintricarboxylic acid, diphtheria toxin, ricin, sodium fluoride, sparsomycin, and trichoderma, but is not limited thereto, and any protein synthesis inhibitor having a similar mechanism may be alternative.

The tetracycline-based antibiotics may include antibiotics selected from the group consisting of doxycycline, minocycline, sarecycline, eravacycline, chlortetracycline, demeclocycline, lymecycline, rolitetracycline, omadacycline, methacycline, oxytetracycline, tetracycline, and tigecycline, and any drug known as a tetracycline-based antibiotic in the art to which the present invention pertains may be substituted.

In another aspect of the present invention, the promoter regulating the expression of the gene encoding the FAF1 protein may be an inducible promoter such as a nitrogen-deficiency inducible promoter or a salt inducible promoter. The inducible promoter may be, for example, a hormone-responsive promoter (*e.g.,* an ecdysone responsive promoter such as that described in US 6,379,945), a metallothionein promoter (US 6,410,828), and a pathogenesis-related (PR) promoter that may react to a chemical such as salicylic acid, ethylene, thiamine, and/or BTH (US 5,689,044), or a combination thereof.

In addition, the inducible promoter may be a light-inducible promoter, a metal inducible promoter, or a temperature (low-temperature or high-temperature) inducible promoter, a nitrogen-deficiency inducible promoter, or an antibiotic-inducible promoter, and may be a promoter known to induce gene expression depending on the presence or absence of light, a metal, a temperature change, a nitrogen deficiency, an antibiotic, or the like in the art to which the present invention pertains. Among these, as the antibiotic-inducible promoter, a thiostrepton-inducible promoter, a tetracycline response element (TRE) promoter, a Plial promoter, and the like, are known, and preferably, the antibiotic-inducible promoter may be a TRE promoter. In addition, the TRE promoter is a promoter that is activated by the tetracycline-based antibiotics described above.

In another aspect of the present invention, the cell line stably expressing the exosome loaded with the FAF1 protein may be a cell line in which the expression of the gene encoding the FAF 1 protein is induced in an environment in which a promoter regulating the expression of the gene encoding the FAF1 protein is activated. In addition, the cell line stably expressing the exosome loaded with the FAF1 protein may be a cell line in which the expression of the gene encoding the FAF1 protein is induced in the presence of a protein synthesis inhibitor.

In an embodiment of the present invention, the transformation of cells using the vector may be performed by treating $1 \times 10^5$ cells to $1 \times 10^7$ cells to be transformed with 1 to 10 $\mu$g of a pTetOne-FAF1 plasmid (Takara Bio Inc., Japan). Preferably, $1 \times 10^6$ cells to be transformed may be cultured by the treatment with 5 $\mu$g of the pTetOne-FAF1 plasmid, but the present invention is not limited thereto.

In another aspect of the present invention, the cell line expressing the exosome loaded with the FAF1 protein may be derived from immune cells, stem cells, somatic cells, plant cell lines, bacterial cell lines, yeast cell lines, mammalian cell lines, or tumor cells.

The immune cells may be selected from the group consisting of dendritic cells, natural killer cells, T cells, B cells, regulatory T cells (Treg cells), natural killer T cells, innate lymphoid cells, macrophages, granulocytes, chimeric antigen receptor-T (CAR-T) cells, lymphokine-activated killer (LAK) cells, and cytokine induced killer (CIK) cells, but are not limited thereto.

The stem cells may be mesoderm stem cells, pluripotent stem cells, multipotent stem cells, or unipotent stem cells, but are not limited thereto. The pluripotent stem cells may be embryonic stem cells (ES cells), embryonic germ cells (EG cells), or induced pluripotent stem cells (iPS cells, iPSCs), but are not limited thereto. The multipotent stem cells may be adult stem cells such as mesenchymal stem cells (derived from adipose, bone marrow, umbilical cord blood, or umbilical cord, *etc.*), a hematopoietic stem cells (derived from bone marrow or peripheral blood), neural stem cells, germ stem cells, and the like, but are not limited thereto. The mesenchymal stem cells (MSC) may be human embryonic stem cell-derived mesenchymal stem cells (hES-MSCs), bone marrow-derived mesenchymal stem cells (BM-MSCs), umbilical cord-derived mesenchymal stem cells (UC-MSCs), or adipose-derived mesenchymal stem cells-conditioned medium (ADSC), but are not limited thereto.

In addition, the stem cells may be autologous or allogeneic stem cells, may be derived from any type of animal including human and non-human mammals, and may be embryonic stem cells, adult stem cells, or induced pluripotent stem cells, but are not limited thereto. The term "embryonic stem cell" refers to a cell extracted in the process of development of an embryo, which is obtained by extracting an inner cell mass from blastocyst embryos just before a fertilized egg is implanted in the uterus of a mother, and culturing the inner cell mass *in vitro.* The embryonic stem cells refer to pluripotent or totipotent cells having capacity for self-renewal that can be differentiated into cells of all tissues of an individual, and in a broad sense, include embryoid bodies derived from the embryonic stem cells.

The somatic cells may be selected from the group consisting of fibroblasts, chondrocytes, synovial cells, keratinocytes, adipocytes, osteoblasts, osteoclasts, and peripheral blood mononuclear cells.

The tumor cells may be derived from human ovarian cancer cell lines (SKOV3 and OVCAR3), human breast cancer cell lines (MCF-7, T47D, BT-474, and MDA-MB-231), human hepatocarcinoma cell lines (Hep3B and HepG2), human glioblastoma cell lines (U87MG and U251), human colorectal cancer cell lines (SW480, HT-29, HCT116, and Caco-2), human lung cancer cell lines (A549, NCIH358, and NCI-H460), a human prostate cancer cell line (22RV1), a human cervical cancer cell line (HeLa), a human melanoma cell line (A375), and a human stomach cancer cell line (NCI-N87), but are not limited thereto.

As the plant cell line, the bacterial cell line, or the yeast cell line, a cell line known to be suitable for protein production in the art, to which the present invention belongs, may be used.

In another aspect of the present invention, the cell line expressing the exosome loaded with the FAF1 protein may be derived from a mammalian cell line. The mammalian cell line may be selected from the group consisting of CHO cell line, NS0 cell line, Sp2/0 cell line, BHK cell line, C127 cell line, HEK293 cell line, HEK293T cell line, HEK-293 STF cell line, 293T/17 cell line, 293T/17 SF cell line, HEK-293.2sus cell line, HEK-293 F cell line, HT-1080 cell line, PER.C6 cell line, NuLi-1 cell line, ARPE-19 cell line, VK2/E6E7 cell line, Ect1/E6E7 cell line, RWPE-2 cell line, WPE-stem cell line, End1/E6E7 cell line, WPMY-1 cell line, NL20 cell line, NL20-TA cell line, WT 9-7 cell line, WPE1-NB26 cell line, WPE-int cell line, RWPE2-W99 cell line, Expi293F cell line, and BEAS-2B cell line, but are not limited thereto. Preferably, the cell line expressing the exosome loaded with the FAF1 protein may be derived from Expi293F cells, which are suspended cells.

In another aspect of the present invention, the cell line stably expressing the exosome loaded with the FAF1 protein may increase the number of exosomes, the exosome-loaded FAF1 protein, and the FAF1 proportion among the exosome-loaded proteins.

In an aspect of the present invention, the cell line stably expressing the exosome loaded with the FAF1 protein may be a cell line in which the amount of exosomes loaded with the FAF1 protein obtained by the cell culture in an environment in which a promoter regulating the expression of the gene encoding the FAF1 protein is activated is increased compared with the amount of exosomes loaded with the FAF1 protein obtained by the cell culture in an environment in which the promoter is inactivated.

In addition, the cell line stably expressing the exosome loaded with the FAF1 protein may be a cell line in which the amount of exosomes loaded with the FAF1 protein obtained by the cell culture in an environment in which a promoter regulating the expression of the gene encoding the FAF1 protein is activated is increased by two times or more, three times or more, four times or more, five times or more, six times or more, seven times or more, eight times or more, nine times or more, 10 times or more, 11 times or more, 12 times or more, 13 times or more, 14 times or more, or 15 times or more compared with the amount of exosomes loaded with the FAF1 protein obtained by the cell culture in an environment in which the promoter is inactivated. Preferably, the cell line stably expressing the exosome loaded with the FAF1 protein may be a cell line in which the amount of exosomes loaded with the FAF1 protein obtained by the cell culture in an environment in which a promoter regulating the expression of the gene encoding the FAF1 protein is activated is increased by three times or more, five times or more, six times or more, or 12 times or more compared with the amount of exosomes loaded with the FAF1 protein obtained by the cell culture in an environment in which the promoter is inactivated.

In another aspect of the present invention, the cell line stably expressing the exosome loaded with the FAF1 protein may be a cell line in which the content of the exosomes loaded with the FAF1 protein by the cell culture in the presence of a protein synthesis inhibitor is increased compared with the content of the exosomes loaded with the FAF 1 protein obtained by the cell culture in the absence of the protein synthesis inhibitor.

In addition, the cell line stably expressing the exosome loaded with the FAF1 protein may be a cell line in which the amount of exosomes loaded with the FAF 1 protein obtained by the cell culture in the presence of a protein synthesis inhibitor is increased by three times or more, four times or more, five times or more, six times or more, seven times or more, eight times or more, nine times or more, 10 times or more, 11 times or more, 12 times or more, 13 times or more, 14 times or more, or 15 times or more compared with the amount of exosomes loaded with the FAF 1 protein obtained by the cell culture in the absence of the protein synthesis inhibitor.

In another aspect of the present invention, the cell line stably expressing the exosome loaded with the FAF 1 protein may be a cell line in which a proportion of the FAF 1 among the proteins in the exosomes is increased compared to a HEK293 cell line transformed with a vector including a gene encoding the FAF1 protein. In addition, the cell line stably expressing the exosome loaded with the FAF1 protein may be a cell line in which a proportion of the FAF1 among the proteins in the exosomes is increased by two times or more, three times or more, four times or more, or five times or more, and preferably three times or more, compared with the HEK293 cell line transformed with the vector including the gene encoding the FAF1 protein.

In still another aspect of the present invention, the cell line stably expressing the exosome loaded with the FAF 1 protein may be a cell line in which the number of exosomes loaded with the FAF 1 protein, the proportion of the FAF1 among proteins in the exosomes, or both thereof are increased compared with the HEK293 cell line transformed with the vector including the gene encoding the FAF 1 protein.

In an aspect of the present invention, the present invention relates to an exosome obtained from a cell line stably expressing the exosome loaded with the FAF 1 protein.

The exosomes may be obtained by a method commonly used in the art to isolate exosomes. The exosome may be extracted by performing size exclusion chromatography, ion exchange chromatography, density gradient centrifugation, differential centrifugation, ultrafiltration, tangential flow filtration, exosome precipitation, a total exosome extraction kit, immune-absorbent capture, affinity method such as affinity capture or affinity purification, immunoassay, microfluidic separation, or a combination thereof.

According to a specific embodiment of the present invention, the culture medium of the cell line stably expressing the exosome loaded with the FAF1 protein may be collected and centrifuged at 300 g for 10 minutes, 2,000 g for 10 minutes, and 10,000 g for 30 minutes to separate the supernatant, the supernatant may be filtered using a 0.2-$\mu$m filter, and may be centrifuged with an ultracentrifuge at 150,000 g for 70 minutes. In addition, the supernatant obtained by the centrifugation may be removed, the pellet may be washed by adding PBS thereto, and then centrifuged again at 150,000 g for 70 minutes and the supernatant may be removed to isolate the exosomes remaining in the lower layer, but the present disclosure is not limited thereto.

In the present invention, the exosomes obtained from the cell line stably expressing the exosome loaded with the FAF 1 protein may be stored at -20 °C to -80 °C.

In addition, in the present invention, the FAF1 proteins may be secreted into the extracellular space via an exosome. The extracellular-secreted FAF1 protein may induce apoptosis in other cells.

In an aspect of the present invention, the present invention may provide a method for preparing a cell line stably expressing an exosome loaded with a FAF1 protein, the method including the steps of: a) transducing a vector including a gene encoding the FAF 1 protein and a promoter regulating the expression of the gene into a cell selected from the group consisting of immune cells, stem cells, somatic cells, plant cells, bacterial cells, yeast cells, mammalian cells or tumor cells to obtain a transformed cell; b) culturing the transformed cell; and c) screening a cell stably expressing the exosome loaded with the FAF 1 protein in the cultured cells. The immune cells, the stem cells, the somatic cells, the tumor cells, the FAF1 protein, the promoter regulating the expression of the gene encoding the FAF1 protein, the vector, and the transformation are the same as described above. In addition, the plant cells, bacterial cells, yeast cells, and mammalian cells refer to cells belonging to or derived from the above-described plant cell line, bacterial cell line, yeast cell line, and mammalian cell line, respectively.

As used herein, the term "culture" refers to a method for growing cells or microorganisms under appropriately artificially controlled environmental conditions. In the present invention, the method for culturing the transformed cells may be performed using a method widely known in the art.

In addition, the medium that may be used for culturing cells in the present invention refers to a known medium used for culturing cells. Examples of the medium may include a medium selected from the group consisting of a commercially produced medium such as Dulbecco's Modified Eagle's Medium (DMEM), Minimal Essential Medium (MEM), Basal Medium Eagle (BME), RPMI 1640, Dulbecco's Modified Eagle's Medium: Nutrient Mixture F-10 (DMEM/F-10), Dulbecco's Modified Eagle's Medium: Nutrient Mixture F-12 (DMEM/F-12), $\alpha$-Minimal essential Medium ($\alpha$-MEM), Glasgow's Minimal Essential Medium (G-MEM), Isocove's Modified Dulbecco's Medium (IMDM), KnockOut DMEM, Essential 8 Medium (E8), or Expi293F expressing medium, or an artificially synthesized medium, or a serum-free medium, a protein-free medium, and a chemically defined medium, which are commercially available, but are not limited thereto. In addition, the medium may include the above-described protein synthesis inhibitor.

In another aspect of the present invention, a method for producing an exosome loaded with a FAF1 protein may include culturing a cell line stably expressing the exosome loaded with the FAF1 protein, and isolating the exosome loaded with the FAF1 protein from the cell line culture medium. The above isolation method may be used for isolating an exosome, preferably, exosome isolation may be performed using a centrifuge.

The expression level of the protein may be used as a marker for selecting cell lines used in the present invention, and the expression of the protein may be measured using a method known in the art. For example, an antibody that specifically detects the protein may be purchased commercially, and may be used to detect the protein by using Western blot, co-immunoprecipitation, immunofluorescence, enzyme-linked immunosorbent assay, and the like. By using such antibodies, an expression level of a specific protein may be specifically identified, but the present invention is not limited thereto.

Hereinafter, the present invention will be described in detail with reference to the following examples.

However, the following examples are only for illustrating the present invention, and the present invention is not limited thereto.

<u>**Modes for the Invention**</u>

**<Example 1> Preparation of Cell Line Capable of Producing FAF1 Exosomes with High Yield**

**Example 1.1. Preparation of FAF1 Exosome Stable Expression Cell Line Derived from Expi293F Cell Line**

**[Cell Culture]** The Expi293F cell line (Gibco, USA) was purchased as base cells for producing a cell line stably producing exosomes loaded with FAF1 proteins (hereinafter, referred to as an FAF1 exosome stable production cell line). It is known that the cells of the Expi293F cell line (hereinafter, Expi293F cells) are suspended cells derived from HEK 293 cell line, which is derived from the human embryonic kidney, can be cultured to high density, are highly transducible, and generate superior protein yields compared to the HEK 293 cells (Thermo Fisher Scientific Expi293F cell line product description, https://www.thermofisher.com/order/catalog/product/A14527). In addition, Expi293F cells may be cultured in Expi293 Expression Medium (Gibco, USA), which is a synthetic medium without serum and external protein, to exclude exosomes or external proteins contained in serum when producing exosomes (Thermo Fisher Scientific Expi293 Expression Medium product description, https://www.thermofisher.com/order/catalog/product/A1435101).

The Expi293F cells were seeded into Expi293 Expression Medium (Gibco, USA) at a concentration of 3 X $10^5$ cells/mL or 4 X $10^5$ cells/mL in Erlenmeyer flask for cell culture and suspended cell culture was performed until the concentration of the cells reached 3 X $10^6$ cells/mL. In this case, under the conditions of 8% $CO_2$, 37 °C, and 125 rpm, the Expi293F cells were maintained by subculture with Expi293 Expression Medium once every 3-4 days.

For transduction, suspended cells were used after adaptation to attached cells. 1 X $10^6$ Expi293F cells were seeded into a 100-mm culture dish and cultured in Dulbecco's modified Eagle's medium (DMEM, WelGENE, Korea) containing 10% Fetal bovine serum (FBS, Atlas Biologicals, USA) and antibiotic-antimycotic (penicillin/streptomycin, Gibco, USA) under the conditions of 37 °C and 5% $CO_2$. After at least 24 hours, it was confirmed that cells were attached to the culture dish and the cell morphology changed (adaptation of the suspended cells to the attached cells).

Meanwhile, the adapted Expi293F cells were maintained in a DMEM medium containing antibiotics, 10% FBS (Atlas Biologicals, USA) and 1% antibiotic-antimycotic (penicillin/streptomycin, Gibco, USA) by subculture once every 3-4 days under the conditions of 5% $CO_2$ and 37 °C.

1 X $10^6$ Expi293F cells adapted to attached cells were seeded into a 100-mm culture dish, and 3 X $10^5$ Expi293F cells adapted to attached cells were seeded into a 60-mm culture dish, and then cultured in Dulbecco's modified Eagle's medium (DMEM, WelGENE, Korea) containing 10% FBS for 24 hours under the conditions of 37 °C and 5% $CO_2$.

**[Transduction]** A pTetOne-FAF1 plasmid was prepared by using a pTetOne plasmid (TaKaRa Bio, Japan) and the FAF1 amino acid sequence of SEQ ID NO: 1, and a pTetOne plasmid (TaKaRa Bio, Japan) without FAF1 was used as a control. The pTetOne-FAF1 plasmid/control plasmid (3 μg or 5 μg), and a puromycin linear marker (100 ng, Takara Bio, Japan) were mixed with an Opti-MEM medium (Thermo Fisher Scientific, USA) to prepare a plasmid-puromycin linear marker-medium mixture having a total volume of 200 μL, and the resulting mixture was reacted at room temperature for 5 minutes. The ExpiFectamine (16 μL, Thermo Fisher Scientific, USA) and the Opti-MEM medium were mixed to prepare a mixture of a total volume of 240 μL, and the resulting mixture was reacted at room temperature for 5 minutes. The plasmid-puromycin linear marker-medium mixture and the ExpiFectamine-medium mixture were mixed and reacted at room temperature for 10 minutes.

The reaction-completed mixture was added to the Expi293F cells cultured in the DMEM medium of the culture dish for 24 hours, and the reaction was performed under the conditions of 5% $CO_2$ and 37 °C for 24 hours to transduce the pTetOne-FAF1 plasmid into the cells.

## Example 1.2. Screening of Cell Lines Stably Producing FAF1 Exosomes

Cells transduced with the pTetOne-FAF1 plasmid prepared in Example 1.1 were cultured in the same culture dish while replacing the DMEM medium containing 10% FBS and puromycin every 2-3 days. Considering the rate of apoptosis, puromycin was added to the medium in an amount at a concentration of 1 μg/mL or 3 μg/mL.

After 1-2 weeks of pTetOne-FAF1 plasmid transduction, non-transduced cells having no puromycin resistance were killed, and when the transduced cells having puromycin resistance formed colonies, the colonies were isolated using Cloning cylinder (Sigma-Aldrich, USA) and cultured in a 35-mm culture dish to obtain 13 cell lines having puromycin resistance (F3-2, F3-3, F3-4, F5-1, F5-2, F5-3, F5-4, F5-5, F5-6, F5-7, F3-1J, F5-2J, and F5-3J).

Then, in order to investigate whether FAF1 was expressed among the 13 cell lines having puromycin resistance by the transduction of TetOne system, doxycycline was added, and then the expression level of FAF1 was measured by Western blot.

Specifically, F3-2, F3-3, F3-4, F5-1, F5-2, F5-3, F5-4, F5-5, F5-6, F5-7, F3-1J, F5-2J, and F5-3J cell lines were each seeded into 60-mm culture dishes by 2 dishes each at 3 X $10^5$, and then cultured in 10% FBS DMEM medium with or without 100 ng/mL of doxycycline for 3 days. The culture medium was replaced once every day in consideration of the half-life (1 day) in the culture medium of doxycycline. After 3 days, the cultured cells were collected, and the expression levels of FAF1 in doxycycline-treated cells were compared with those of cells not treated with doxycycline by Western blot.

In order to perform Western blot, F3-2, F3-3, F3-4, F5-1, F5-2, F5-3, F5-4, F5-5, F5-6, F5-7, F3-1J, F5-2J, and F5-3J cell culture media were each centrifuged at 300 g for 10 minutes, and then the supernatant was removed to obtain cell pellets. A mammalian lysis buffer (80 μL) was added to the cell pellets, and the cells were dissolved on ice for 30 minutes. The proteins in the cell pellets were diluted with a Bradford solution, and then the concentration thereof was measured at a wavelength of 595 nm of a spectrophotometer (MECASYS, Korea). A certain amount of the pellets dissolved so as to reach 20 μg of protein was mixed with 5 μL of SDS sample buffer, and reacted in boiling water for 3 minutes. A 10% acrylamide gel was made using a Western blot kit (Amersham Biosciences, UK), and a protein sample of cells boiled for 3 minutes was placed on the gel in a certain amount, and the protein was separated by size by applying a voltage of 80-100 V. The acrylamide gel containing the separated protein was overlapped with a nitrocellulose membrane (GE Healthcare, USA) and a current of 200 mA for 2 hours was applied thereto to transfer the protein to the nitrocellulose membrane. The protein-transferred nitrocellulose membrane was blocked with skimmed milk, anti-FAF1 antibodies (E-4, Santacruz, 1:1000, mouse IgGs) were attached, then the secondary antibodies (HRP-anti-mouse, 31439, Thermo Fisher Scientific, 1:1000, Goat IgGs) were attached again, and the amount of the FAF1 protein was confirmed by the Western blot detection kit (AbFrontier, Korea) and the ChemiDoc-It Imaging System (UVP, USA), and the Western blot result was quantified by the ImageJ. B-actin and GAPDH were used as a loading control.

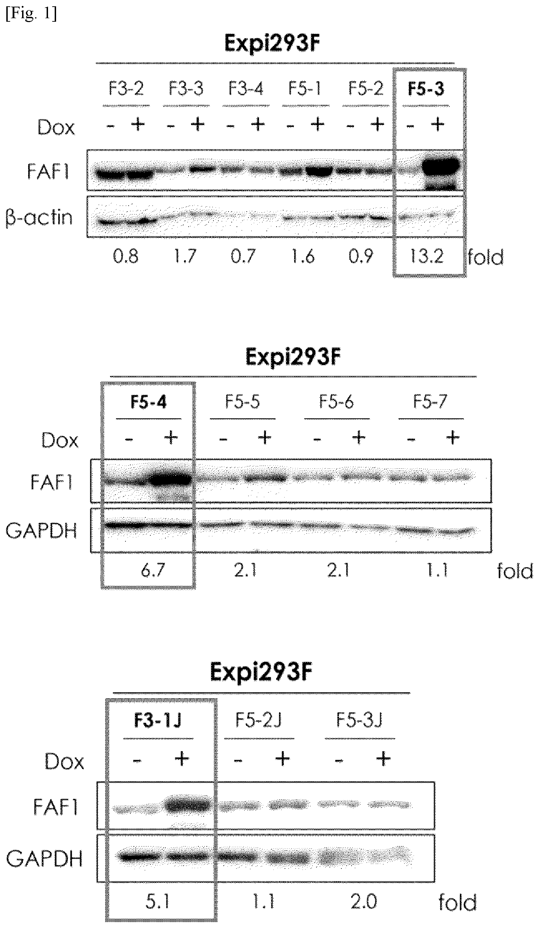

Cells that produce FAF1 three times or more compared to cells not treated with doxycycline were used as the selection criteria for FAF1 exosome stable production cell lines, and three types of cells (F5-3, F5-4, and F3-1J) met the selection criteria for the FAF1 exosome stable production cell lines (FIG. 1). Compared to cells not treated with doxycycline, F5-3 produced 13.2-fold FAF1, F5-4 produced 6.7-fold FAF1, and F3-1J produced 5.1-fold FAF1. For F5-3, F5-4, and F3-1J cell lines, 1 X $10^7$ cells were maintained by subculture in the Expi293 expression medium (Gibco, USA) in the Erlenmeyer flask for cell culture under the conditions of 8% $CO_2$, 37 °C, and 125 rpm once every 3-4 days, and adapted into suspended cells, thereby establishing on/off-capable FAF1 exosome stable production cell lines.

## Example 1.3. Cell Viability and Doubling Time of On/off-capable FAF1 Exosome Stable Expression Cell Lines

On/off-capable FAF1 exosome stable expression cell lines, F5-3, F5-4, and F3-1J, were subcultured under the conditions of Example 1.2 above. In these FAF1 exosome stable expression cell lines, when the cell division rate was slow, the cell concentration seeded into the Erlenmeyer flask for cell culture was set to 6 X $10^5$ cells/mL or 8 X $10^5$ cells/mL, and

thus the culture was performed under the conditions of Example 1.2. so that the cell concentration could reach the concentration of 3 X $10^6$ cells/mL within 3-4 days.

The Expi293F cell line was maintained by subculture once every 3-4 days under the conditions of 8% $CO_2$, 37 °C, and 125 rpm in an Expi293 expression medium (Gibco, USA).

The subculture cell culture medium (10 μL) was separated, mixed with 140 μL of Muse Count & Viability Kit (Luminex, USA), and reacted at room temperature for 5 minutes. Then, the mixture was added to the Muse cell analyzer (Luminex, USA), and the number of cells and viability were measured.

A doubling time was calculated using the following equation using the number of cells measured during the subculture.

$$Doubling\ Time = \frac{T}{lnC - lnC_0} \times ln2$$

T = culture time
C = cell concentration after culture
$C_0$ = cell concentration before culture

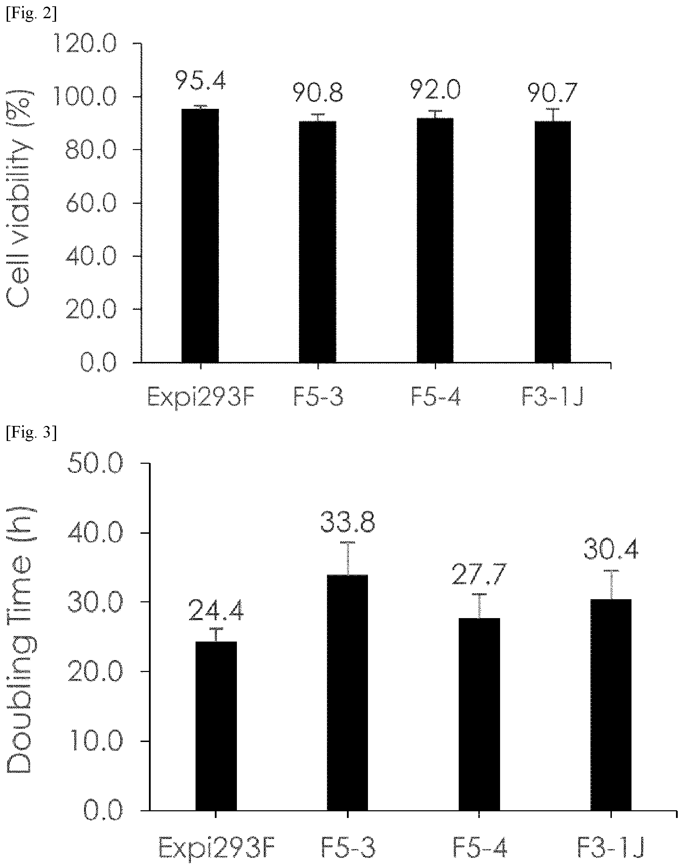

Viabilities and cell concentrations were measured by the Muse cell analyzer while subculturing Expi293F cells, F5-3 cells, F5-4 cells and F3-1J cells at least 10 times (FIGS. 2 and 3). The average viability of each cell was measured as 95.4±1.12% of Expi293F cells, 90.8±2.63% of F5-3 cells, 92.0±2.64% of F5-4 cells, and 90.7±4.53% of F3-1J cells. The viability of the prepared FAF1 exosome stable expression cell line was slightly decreased compared to the Expi293F cell line, but all the cell lines showed a good viability of 90% or higher.

When the doubling time of each cell was calculated using the measured cell concentration, the Expi293F cells were measured to be 24.4±1.75 hours, the F5-3 cells were measured to be 33.8±4.76 hours, the F5-4 cells were measured to be 27.7±3.40 hours, and the F3-1J cells were measured to be 30.4±4.17 hours. The prepared FAF1 exosome stable expression cell line had a slightly increased doubling time compared with the Expi293F cell line. It is assumed that showing the low viability and high doubling time compared to the original Expi293F cell line was because the transduced TetOne-FAF1 system did not completely suppress the expression of FAF1 inducing apoptosis.

**<Example 2> Characterization of Exosomes Derived from FAF1 Exosome Stable Expression Cell Line**

**Example 2.1. Isolation of Exosomes Using Ultracentrifuge**

The exosomes of the cell line stably expressing FAF1 and the Expi293F cell line were isolated using an ultracentrifuge (Optima XE-100, BECKMAN Coulter, USA).

Specifically, Expi293F, F5-3, F5-4 and F3-1J cells having a concentration of 3 X $10^6$ cells/mL were each cultured in 50 mL of Expi293 expression medium containing 1 μg/mL of doxycycline for 48 hours or 72 hours, and the medium was not replaced in the middle in order to isolate all exosomes within the culture period. The medium of each cell was collected and centrifuged at 300 g for 10 minutes, 2,000 g for 10 minutes, and 10,000 g for 30 minutes to separate the supernatant, and the supernatant was filtered with a 0.2-μm syringe filter (BioFACT, Korea).

To isolate the exosomes for use in the experiment, the supernatant was centrifuged at 150,000 g for 70 minutes with a 45 Ti rotor in the BECKMAN Coulter optima XE-100 ultracentrifuge to discard the supernatant and wash the pellet with PBS. The washed PBS was centrifuged again at 150,000 g for 70 minutes, the supernatant was then discarded, and the remaining was collected in an EP tube using PBS.

The isolated exosomes were stored at -80 °C, and used within one week after the isolation or stored at 4 °C after thawing for use.

**Example 2.2. Confirmation of FAF1 Exosome Stable Expression Cells and Loading of FAF1 and Exosome-related Markers in Isolated Exosomes**

To investigate whether the exosomes isolated from the FAF1 exosome stable expression cell line of Example 1.2 above are the same as the exosomes isolated from the Expi293F cell line, which is the parent cell, the amounts of proteins were compared, by a Western blot method, between the Expi293F cells and the exosomes isolated therefrom and the F5-3 cells showing the highest FAF1 expression level in the Western blot experiment of Example 1.3., and the exosomes isolated therefrom.

Specifically, the cells and exosomes were each isolated by the method of Example 2.1. in a control group in which the Expi293F cells and F5-3 cells were cultured for 48 hours without doxycycline, and experimental groups in which 1 μg/mL of doxycycline was treated and cultured for 48 hours or 72 hours.

In order to perform the Western blot, the cell culture medium was centrifuged at 300 g for 10 minutes to obtain cell pellets from which the supernatant was removed. A mammalian lysis buffer (50 μL) was added to the cell pellets, and the cell pellets were dissolved on ice for 30 minutes.

The dissolved pellets were mixed with 5 μL of SDS sample buffer, and reacted in boiling water for 3 minutes. With respect to the exosomes isolated in Example 2.1., 5 μL of SDS sample buffer and the exosomes were mixed and reacted in boiling water for 3 minutes. An 8-10% acrylamide gel was prepared using the Western blot kit (Amersham Biosciences, UK). In order to perform the Western blot of the same amount of protein, the intracellular proteins were diluted with a Bradford solution, and then the concentration was measured at a wavelength of 595 nm of the spectrophotometer (MECASYS, Korea).

The concentration of exosomes was measured using Nanoparticle tracking analysis (NTA), and the isolated exosomes were diluted at a concentration of $1 \times 10^8$ particles/mL to $1 \times 10^9$ particles/mL, the concentration of exosomes was measured using NS300 (Malvern Panalytical, UK), and then $5 \times 10^9$ exosomes were placed on the acrylamide gel. After boiling for 3 minutes, samples of cells and exosome proteins were put on the gel in a certain amount, and a voltage of 80-100 V was applied to separate the proteins by size. The acrylamide gel containing the separated proteins was placed to overlap a nitrocellulose membrane and a current of 200 mA was applied for 2 hours to transfer the proteins to the nitrocellulose membrane. The nitrocellulose membrane to which the proteins were transferred was blocked with skimmed milk, the primary antibodies of Table 1 below were attached thereto, then the secondary antibodies of Table 1 below were attached thereto, and the amounts of FAF1 and exosome-related marker proteins (Alix, CD81, CD47, CD9 and Syntenin-1) were confirmed by the western blot detection kit (AbFrontier, Korea) and ChemiDoc-It Imaging System (UVP, USA), and GAPDH was used as a cell loading control.

[Table 1]

|  | Primary antibody | Dilution |
|---|---|---|
| Anti-FAF1 | E-4, Santacruz | 1:1000, mouse IgG |
| Anti-β-actin | A5316, Sigma-Aldrich | 1:5000, mouse IgG |
| Anti-GAPDH | LF-PA0212, AbFrontier | 1:10000, rabbit IgG |
| Anti-Alix | 1A12, Santacruz | 1:1000, mouse IgG |
| Anti-CD81 | B-11, Santacruz | 1:1000, mouse IgG |
| Anti-CD47 | PA5-114984, Thermo Fisher Scientific | 1:1000, rabbit IgG |
| Anti-CD9 | C-4, Santacruz | 1:100, mouse IgG |
| Anti-Syntenin-1 | Ab133267, Abcam | 1:5000, rabbit IgG |
|  | Secondary antibody | Dilution |
| HRP-anti-mouse | 31439, Thermo Fisher Scientific | 1:1000, Goat IgG |
| HRP-anti-rabbit | A16124 Thermo Fisher Scientific | 1:1000, Goat IgG |

The cellular proteins (10 or 20 μg) were added depending on the kind of the antibody to be used to perform Western blotting. When the amount of FAF1 loaded in the exosomes was quantified by Western blot, a predetermined amount of recombinant human FAF1 protein (BOSTER, USA) was subjected to Western blot together and measured with ImageJ, and the measured value was compared with the values measured of FAF1 in the exosomes.

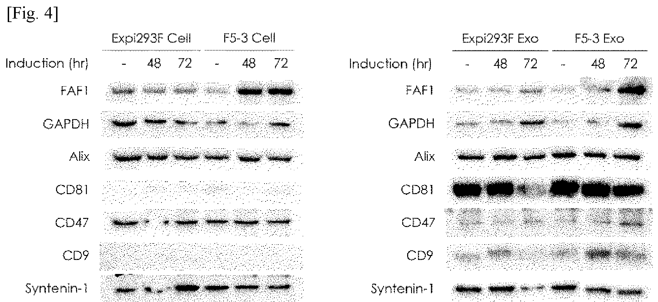

The intracellular FAF1 was significantly increased only when the F5-3 cells were treated with doxycycline, and the amount of FAF1 loaded in the exosomes was also significantly increased when the F5-3 cells were treated with doxycycline (FIG. 4, F5-3 Cell, and F5-3 Exo). In particular, when the cells were cultured for 72 hours, the largest amount of FAF1 loaded was shown. All exosome-related markers were present in the exosomes, and thus it was confirmed that the proteins were proteins in the exosomes.

**Example 2.3. Comparison of the Number of Exosomes between HEK293 Cell Line and FAF1 Exosome Stable Expression Cell Line, and Loaded Amount of FAF1**

Differences in the yields of FAF1-loaded exosomes of HEK293 cells which are transduced with 3x Flag tag-FAF1 plasmid/control plasmid and cultured for 24 hours, Expi293F cells which are cultured for 48 hours without doxycycline, Expi293F cells which are treated with 1 $\mu$g/mL of doxycycline and cultured for 48 hours, F5-3 cells which are cultured for 48 hours without doxycycline, and F5-3 cells which are treated with 1 $\mu$g/mL of doxycycline and cultured for 48 hours were compared.

The HEK293 cells were maintained in a DMEM medium containing 10% FBS and antibiotics by subculture once every 3-4 days under the conditions of 5% $CO_2$ and 37 °C.

To temporarily overexpress FAF1 in the HEK293 cells, 3x Flag tag-FAF1 plasmid/control plasmid (8 $\mu$g) was transduced into 6.5 X $10^6$ HEK293 cells. Specifically, the HEK293 cells were seeded into a 150-mm culture dish, and then were cultured in the DMEM medium containing 10% FBS for 24 hours under the conditions of 5% $CO_2$ and 37 °C. The 3x Flag tag-FAF1 was prepared by the previously known method (Yu et al., FAF1 mediates regulated necrosis through PARP1 activation upon oxidative stress leading to dopaminergic neurodegeneration. Cell Death & Differentiation, 2016, 23.11: 1873-1885). DNA:BioT (Bioland scientific, USA) (a ratio of 1:1.5) was mixed in the DMEM medium without adding FBS and antibiotics to form a mixture of 180 $\mu$L in total, and the mixture was reacted at room temperature for 5 minutes. The mixture was added to the cells cultured for 24 hours, and reacted under the conditions of 5% $CO_2$ and 37 °C for 24 hours.

To isolate the exosomes, the medium of the transduced HEK293 cells was replaced with a DMEM medium without FBS and antibiotics, and the cells were cultured for 24 hours. When the HEK293 cells in which FAF1 was transiently overexpressed were cultured without FBS for a long period of time, apoptosis occurred, and thus the cells were cultured only for 24 hours, and exosomes were isolated by the method of Example 2.1.

The Expi293F cells and F5-3 cells were cultured in Expi293 expression medium, or in Expi293 expression medium containing 1 $\mu$g/mL of doxycycline.

The amount of FAF1 loaded in the exosomes isolated from each of the HEK293, Expi293F, and F5-3 cells was quantified by Western blot and ELISA.

When the exosomes were quantified by the Western blot method of Example 2.2, recombinant FAF1 proteins in which the amounts of proteins were accurately measured were loaded together to measure the protein band by ImageJ and then compare and quantify the protein band.

ELISA analysis was performed using Human FAF1 ELISA Kit (Abbexa, UK). A mammalian lysis buffer (10 $\mu$L) was added to the exosomes isolated from each of the HEK293, Expi293F, and F5-3 cells, and the exosomes were dissolved on ice for 30 minutes. Then, a standard/sample diluent was added to dilute the exosomes so that 1 X $10^8$ or 1 X $10^7$ HEK293 exosomes per one well of 96-well plate of the ELISA Kit were added to the well, and 1 X $10^9$ or 1 X $10^8$ Expi293F and F5-3 exosomes were added. FAF1 standards were diluted to be concentrations of 50, 25, 12.5, 6.25, 3.13, 1.56 and 0.78 ng/mL, and the FAF1 standards and the diluted exosomes together were seeded by 100 $\mu$L each into the 96-well plate of the ELISA Kit, and reacted at 37 °C for 2 hours. The supernatant was discarded, and A solution in the ELISA Kit was diluted into a total solution, seeded thereto by 100 $\mu$L each, and reacted at 37 °C for 1 hour. After the supernatant was discarded, a wash buffer was added thereto and washed twice. The supernatant was discarded, and B solution in the ELISA Kit was diluted into a solution for B solution, seeded thereto by 100 $\mu$L each, and reacted at 37 °C for 1 hour. After the supernatant was discarded, a wash buffer was added thereto and washed three times. After the supernatant was discarded, the TMB solution was seeded thereto by 90 $\mu$L each, reacted at 37 °C for 20-30 minutes, and a stop solution was seeded thereto by 50 $\mu$L each. Each well of which the color was changed according to the amount of proteins was measured at a wavelength of 450 nm of a microplate reader (PerkinElmer, USA) to quantify the FAF1 protein. The average value was derived by combining the measured values, which are the amount of FAF1 loaded and the number of exosomes measured by the Western blot and ELISA.

In addition, a proportion of FAF1 in the total proteins of one exosome was measured. The exosome proteins were dissolved by the mammalian lysis buffer, exposed in the exosomes, then diluted with a Bradford solution, and the amount of proteins was measured at a wavelength of 595 nm of the spectrophotometer (MECASYS, Korea).

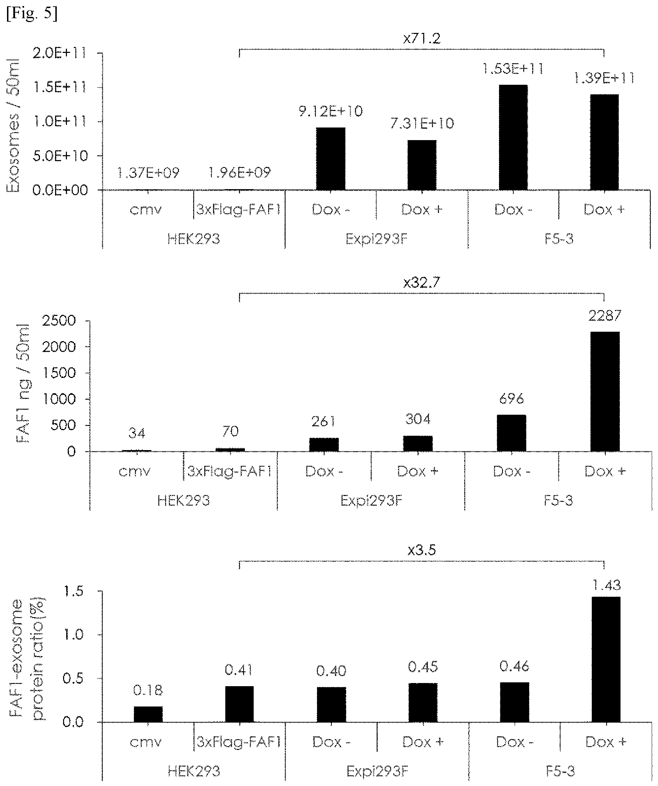

FIG. 5 shows the loaded amount of FAF1, the measured value of the number of exosomes, and the proportion of FAF1 in the total proteins of a single exosome.

In terms of the number of exosomes produced based on 50 mL of the culture medium, the Expi293F and F5-3 cells were at least 3.7 times greater than the HEK293 cells. This means that when the same culture medium is used, the Expi293F and F5-3 cells may be cultured more densely, thereby producing more exosomes at one time. When comparing the yields of FAF1-loaded exosomes, the F5-3 cells, which were induced by doxycycline, produced exosomes 71.2 times greater than the HEK293 cells.

Based on 50 mL of the culture medium, the total amount of the FAF1 protein loaded on the exosome was measured as 70 ng in the HEK293 exosome and 2,287 ng in the F5-3 exosome, and it was confirmed that the F5-3 cells produced FAF1 proteins 32.7 times greater than the HEK293 cells. This indicates that the loaded amount of FAF1 per exosome is less for the F5-3 cells than for the HEK293 cells, but the total loaded amount of FAF1 that may be produced at one time when using the same volume of culture medium is 32.7 times more for the F5-3 cells than for the HEK293 cells, and that more FAF1 may be delivered to the target.

When comparing the amount of FAF1 protein measured in each cell with the amount of exosome proteins, 0.41% was measured in the HEK293 exosomes transduced with 3x Flag tag-FAF1, and 1.43% was measured in the F5-3 exosomes cultured for 48 hours after the treatment with 1 μg/mL of doxycycline. This indicates that the exosomes isolated from the HEK293 cells contain more impurities than the F5-3 because not only FAF1 proteins but also other various proteins exist.

Therefore, it was confirmed that the exosomes isolated from the F5-3 cells produced more FAF1 proteins and more exosomes with higher purity when using the same volume of culture medium.

**Claims**

1. A cell line stably expressing an exosome loaded with a FAF1 protein.

2. The cell line of claim 1, wherein the cell line is transformed with a vector comprising a gene encoding the FAF1 protein and a promoter regulating the expression of the gene.

3. The cell line of claim 2, wherein the expression of the gene encoding the FAF1 protein is induced in an environment in which the promoter regulating the expression of the gene is activated.

4. The cell line of claim 2, wherein the FAF1 protein comprises the amino acid sequence of SEQ ID NO: 1.

5. The cell line of claim 2, wherein the promoter regulating the expression of the gene encoding the FAF1 protein is operated by a protein synthesis inhibitor.

6. The cell line of claim 5, wherein the protein synthesis inhibitor is selected from the group consisting of aminoglycoside antibiotics, tetracycline-based antibiotics, macrolide antibiotics, lincosamide antibiotics, streptogramin antibiotics, pleuromutilin antibiotics, phenicol antibiotics, fusidic acid antibiotics, oxazolidinone antibiotics, anisomycin antibiotics, edeine antibiotics, pactamycin antibiotics, puromycin antibiotics, cyclohexamide, aurintricarboxylic acid, diphtheria toxin, ricin, sodium fluoride, sparsomycin, and trichoderma.

7. The cell line of claim 6, wherein the tetracycline-based antibiotics are selected from the group consisting of doxycycline, minocycline, sarecycline, eravacycline, chlortetracycline, demeclocycline, lymecycline, rolitetracycline, omadacycline, methacycline, oxytetracycline, tetracycline, and tigecycline.

8. The cell line of claim 5, wherein the promoter regulating the expression of the gene encoding the FAF1 protein is a tetracycline response element (TRE) promoter.

9. The cell line of claim 2, wherein a vector comprising a linear puromycin marker gene is further used during transformation.

10. The cell line of claim 1, being derived from immune cells, stem cells, somatic cells, mammalian cell line, or tumor cells.

11. The cell line of claim 10, wherein the mammalian cell line is a cell line selected from the group consisting of CHO cell line, NS0 cell line, Sp2/0 cell line, BHK cell line, C127 cell line, HEK293 cell line, HEK293T cell line, HEK-293 STF cell line, 293T/17 cell line, 293T/17 SF cell line, HEK-293.2sus cell line, HEK-293 F cell line, HT-1080 cell line, PER.C6 cell line, NuLi-1 cell line, ARPE-19 cell line, VK2/E6E7 cell line, Ect1/E6E7 cell line, RWPE-2 cell line, WPE-stem cell line, End1/E6E7 cell line, WPMY-1 cell line, NL20 cell line, NL20-TA cell line, WT 9-7 cell line, WPE1-NB26 cell line, WPE-int cell line, RWPE2-W99 cell line, Expi293F cell line, and BEAS-2B cell line.

12. The cell line of claim 10, wherein the mammalian cell line is an Expi293F cell line.

13. The cell line of claim 2, wherein the amount of the exosome loaded with the FAF1 protein obtained by the cell culture in an environment in which a promoter regulating the expression of the gene encoding the FAF1 protein is activated is increased by 2 times to 15 times greater than the amount of the exosome loaded with the FAF1 proteins obtained by the cell culture in an environment in which the promoter is inactivated.

14. The cell line of claim 1, wherein a proportion of FAF1 among the proteins in the exosomes is increased by at least three times greater than the HEK293 cell line transformed with the vector comprising the gene encoding the FAF1 protein.

15. The cell line of claim 1, wherein the number of exosomes loaded with the FAF1 protein, the proportion of the FAF1 among the proteins in the exosomes, or both thereof are increased compared with the HEK293 cell line transformed with the vector comprising the gene encoding the FAF1 protein.

16. A method for preparing a cell line stably expressing an exosome loaded with a FAF 1 protein, the method comprising the steps of:

a) transducing a vector comprising a gene encoding the FAF1 protein and a promoter regulating the expression of the gene into a cell selected from the group consisting of immune cells, stem cells, somatic cells, plant cells, bacterial cells, yeast cells, mammalian cells or tumor cells to obtain a transformed cell;
b) culturing the transformed cell; and
c) screening cells stably expressing the exosome loaded with the FAF1 protein in the cultured cells.

17. A method for producing an exosome loaded with the FAF 1 protein, the method comprising the steps of:

a) culturing the cell line according to any one of claims 1 to 15; and
b) isolating the exosome loaded with the FAF1 protein in the cell line culture medium.

18. The method of claim 17, wherein step b) is performed by using size exclusion chromatography, ion exchange chromatography, density gradient centrifugation, differential centrifugation, ultrafiltration, tangential flow filtration, exosome precipitation, a total exosome extraction kit, immune-absorbent capture, affinity capture, affinity purification, immunoassay, microfluidic separation, or a combination thereof.

[Fig. 1]

[Fig. 2]

[Fig. 3]

[Fig. 4]

[Fig. 5]

## INTERNATIONAL SEARCH REPORT

| International application No. |
| --- |
| **PCT/KR2023/014695** |

### A. CLASSIFICATION OF SUBJECT MATTER

**C12N 15/85**(2006.01)i; **C07K 14/47**(2006.01)i; **C12N 15/63**(2006.01)i; **C12N 5/073**(2010.01)i

According to International Patent Classification (IPC) or to both national classification and IPC

### B. FIELDS SEARCHED

Minimum documentation searched (classification system followed by classification symbols)

C12N 15/85(2006.01); A61K 38/16(2006.01); A61K 47/68(2017.01); A61K 47/69(2017.01); C12N 15/63(2006.01); C12N 15/67(2006.01); G01N 33/68(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: FAF1(Fas-associated factor 1), 엑소좀(exosome), 세포주(cell lines), 암 (cancer), 프로모터(promoter), 벡터(vector)

### C. DOCUMENTS CONSIDERED TO BE RELEVANT

| Category* | Citation of document, with indication, where appropriate, of the relevant passages | Relevant to claim No. |
| --- | --- | --- |
| X | KR 10-2049199 B1 (THE INDUSTRY & ACADEMIC COOPERATION IN CHUNGNAM NATIONAL UNIVERSITY (IAC)) 26 November 2019 (2019-11-26)<br>See claims 1-7; and paragraphs [0046], [0049]-[0050] and [0077]-[0078]. | 1-2,10-12,14-18 |
| Y |  | 3-9,13 |
| Y | KR 10-2004-0072643 A (TOUDAI TLO, LTD.) 18 August 2004 (2004-08-18)<br>See claims 1, 11-12 and 22-23; paragraphs [0079] and [0087]; and example 11. | 3,5-9,13 |
| Y | NCBI. GenBank Accession No. NP_008982.1. FAS-associated factor 1 [Homo sapiens]. 19 November 2020.<br>See entire document. | 4 |
| A | KR 10-2018-0078173 A (KOREA INSTITUTE OF SCIENCE AND TECHNOLOGY) 09 July 2018 (2018-07-09)<br>See claims 1-7. | 1-18 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **08 January 2024** | **09 January 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
|---|
| **PCT/KR2023/014695** |

### C. DOCUMENTS CONSIDERED TO BE RELEVANT

| Category* | Citation of document, with indication, where appropriate, of the relevant passages | Relevant to claim No. |
|---|---|---|
| A | KR 10-2004-0101707 A (PAI CHAI HAKDANG) 03 December 2004 (2004-12-03)<br>See entire document. | 1-18 |

Form PCT/ISA/210 (second sheet) (July 2022)

**INTERNATIONAL SEARCH REPORT**

International application No.

**PCT/KR2023/014695**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

International application No.

**PCT/KR2023/014695**

| Patent document cited in search report | | | Publication date (day/month/year) | Patent family member(s) | | | Publication date (day/month/year) |
|---|---|---|---|---|---|---|---|
| KR | 10-2049199 | B1 | 26 November 2019 | US | 2022-0214361 | A1 | 07 July 2022 |
| | | | | WO | 2020-235757 | A1 | 26 November 2020 |
| KR | 10-2004-0072643 | A | 18 August 2004 | AT | 483805 | T | 15 October 2010 |
| | | | | AU | 2002-343792 | A1 | 10 June 2003 |
| | | | | AU | 2002-354121 | A1 | 10 June 2003 |
| | | | | CA | 2468955 | A1 | 05 June 2003 |
| | | | | CN | 100500854 | C | 17 June 2009 |
| | | | | CN | 1617929 | A | 18 May 2005 |
| | | | | EP | 1462525 | A1 | 29 September 2004 |
| | | | | EP | 1462525 | B1 | 06 October 2010 |
| | | | | HK | 1078105 | A1 | 03 March 2006 |
| | | | | JP | 3642573 | B2 | 27 April 2005 |
| | | | | US | 2004-0002077 | A1 | 01 January 2004 |
| | | | | US | 2005-0048647 | A1 | 03 March 2005 |
| | | | | US | 2005-0197315 | A1 | 08 September 2005 |
| | | | | WO | 03-046173 | A1 | 05 June 2003 |
| | | | | WO | 03-046186 | A1 | 05 June 2003 |
| KR | 10-2018-0078173 | A | 09 July 2018 | CN | 110248645 | A | 17 September 2019 |
| | | | | CN | 110248645 | B | 26 April 2022 |
| | | | | EP | 3563835 | A1 | 06 November 2019 |
| | | | | JP | 2020-504613 | A | 13 February 2020 |
| | | | | JP | 6857736 | B2 | 14 April 2021 |
| | | | | US | 11319360 | B2 | 03 May 2022 |
| | | | | US | 2020-0148746 | A1 | 14 May 2020 |
| | | | | US | 2022-0251170 | A1 | 11 August 2022 |
| | | | | WO | 2018-124835 | A1 | 05 July 2018 |
| KR | 10-2004-0101707 | A | 03 December 2004 | KR | 10-0692416 | B1 | 09 March 2007 |

Form PCT/ISA/210 (patent family annex) (July 2022)

**EP 4 596 699 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 102053065
- KR 1020180078173
- US 6379945 B
- US 6410828 B
- US 5689044 A

### Non-patent literature cited in the description

- **XIE, FENG et al.** FAF1 phosphorylation by AKT accumulates TGF-B type II receptor and drives breast cancer metastasis.. *Nature communications*, 2017, vol. 8 (1), 1-16

- **YU et al.** FAF1 mediates regulated necrosis through PARP1 activation upon oxidative stress leading to dopaminergic neurodegeneration. *Cell Death & Differentiation*, 2016, vol. 23 (11), 1873-1885